(19) Europäisches Patentamt
European Patent Office
Office européen des brevets

(11) Numéro de publication: **0 012 643**
**B1**

# (12) FASCICULE DE BREVET EUROPEEN

(45) Date de publication du fascicule de brevet: **17.02.82**

(21) Numéro de dépôt: **79400890.4**

(22) Date de dépôt: **20.11.79**

(51) Int. Cl.³: **C 07 D 211/32**, C 07 D 211/22, C 07 D 211/18, C 07 D 211/12, C 07 D 211/26, C 07 D 211/24, C 07 D 211/28, A 61 K 31/445, C 07 D 213/50, C 07 D 213/26

(54) **Dérivés de la phényl-1 (pipéridyl-4)-3 propanone-1, procédés pour leur préparation, et médicaments les contenant.**

(30) Priorité: **05.12.78 FR 7834185**

(43) Date de publication de la demande: **25.06.80 Bulletin 80/13**

(45) Mention de la délivrance du brevet: **17.02.82 Bulletin 82/7**

(84) Etats contractants désignés: **BE CH DE FR GB IT LU NL SE**

(56) Documents cités:
**GB-A-395 231**
**US-A-3 637 712**
**JOURNAL OF THE CHEMICAL SOCIETY, (C), 1969,**
**K. B. PRASAD et al.: »Syntheses of pyridine alkaloids and related compounds. Part II. Syntheses of some 4-alkyl and 4 (1 hydroxyalkyl)-piperidines«,**
**pages 2134-2136.**

(73) Titulaire: **PHARMINDUSTRIE, 35 Quai du Moulin de Cage, F-92231 Gennevilliers (FR)**

(72) Inventeur: **Champseix, Alain André, 12, rue Hector Berlioz, F-91470 Forges les Bains (FR)**
Inventeur: **Le Fur, Gérard Roger, 35, rue du Progrès, F-92350 Plessis Robinson (FR)**

(74) Mandataire: **Houssin, Jean, PRODUITS CHIMIQUES UGINE KUHLMANN Service Propriété Industrielle Tour Manhattan Cedex 21, F-92087 Paris La Defense 2 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



BUNDESDRUCKEREI BERLIN

Dérivés de la phényl-1 (pipéridyl-4)-3 propanone-1, procédés pour leur préparation et médicaments les contenant

La présente invention concerne de nouveaux médicaments, utiles notamment comme antiarythmiques et comme psychotropes, en particulier pour le traitement des états de dépression et d'anxiété, contenant en tant qu'agent actif un dérivé de la phényl-1 (pipéridyl-4)-3 propanone-1 répondant à la formule (I) ci-dessus ou un sel d'un tel composé avec un acide pharmaceutiquement acceptable.

Les dérivés de la phényl-1 (pipéridyl-4)-3 propanone-1 incorporés en tant qu'agent actif dans les médicaments selon l'invention peuvent être représentés par la formule:

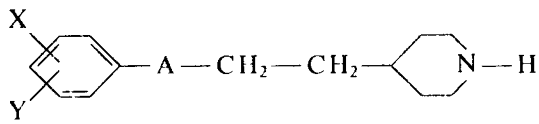

(I)

dans laquelle X et Y sont identiques ou différents et représentent des atomes d'hydrogène ou d'halogène (chlore, fluor, brome, iode) ou des groupes alkyle ayant 1 à 4 atomes de carbone, alcoxy ayant 1 à 4 atomes de carbone, alkylthio ayant 1 à 4 atomes de carbone, trifluorométhyle, hydroxy, amino, monoalkylamino ayant 1 à 4 atomes de carbone, ou amino substitué par un groupe alkylsulfonyle ayant 1 à 4 atomes de carbone, par un groupe alkylcarbonyle ayant 1 à 5 atomes de carbone, en particulier acétyle, ou par un groupe benzoyle, et A représente un groupe

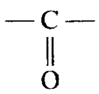

CHOH ou $CH_2$.

Les composés de formule (I) pour lesquels A est $CH_2$, Y est un atome d'hydrogène et X est un atome d'hydrogène ou un groupe p-méthoxy, m-méthoxy, o-méthoxy, p-méthyle, p-isopropyle ou p-tert-butyle sont déjà connus. Ils ont été décrits en tant qu'intermédiaires de synthèse (cf. DOS 2 456 947; brevet belge n° 818 260; K B. PRASAD et Coll., J. Chem. Soc. (C), 1969, 2134–2136; E. O. MAGARIAN et Coll., J. Pharm. Sci. 62, 325 [1973]), mais aucun de ces composés n'a été jusqu'à présent préconisé comme médicament. Il est en outre connu, par le brevet US-A-3 637 712, des aryl-1 [(N-alkyl) pipéridyl-2]-3 propanols ou propanones dont certains ont la propriété d'empêcher les tremblements ou ont une activité hypotensive.

Les composés de formule (I) autres que ceux cités dans le paragraphe précédent sont nouveaux et font partie en tant que tels de l'invention.

Les produits de formule générale (I) pour lesquels A représente le groupe $CH_2$ peuvent être préparés par réduction des produits correspondants de formule (I) pour lesquels A représente le groupe CO ou de leurs dérivés N-benzoylés (la coupure du groupement benzoyle s'effectuant dans les conditions de la réduction). Pour cette réduction on utilise les méthodes connues en soi qui permettent de transformer un groupe CO en un groupe $CH_2$, par exemple celles décrites par R. B. WAGNER et H. D. ZOOK, Synthetic Organic Chemistry, p. 5 (J. Wiley and Sons – 1953).

On utilise avantageusement comme agent réducteur l'hydrate d'hydrazine en présence d'un hydroxyde de métal alcalin tel que l'hydroxyde de sodium, au sein d'un solvant tel qu'un alcool. On opère généralement à la température d'ébullition du solvant.

Les produits de formule générale (I) pour lesquels A représente le groupement $CH_2$ peuvent également être préparés par hydrogénation catalytique des composés pyridiniques de formule:

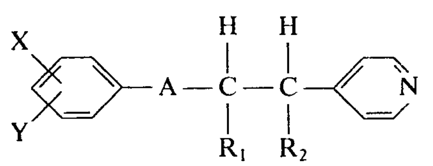

(II)

ou de leurs sels.

Dans la formule (II), A, X et Y ont les mêmes significations que dans la formule (I), $R_1$ représente un atome d'hydrogène, $R_2$ représente un atome d'hydrogène ou un groupe OH, $R_1$ et $R_2$ pouvant en outre former ensemble une liaison simple.

Les produits de formule (II) utilisés de préférence parce que les plus accessibles sont les suivants:

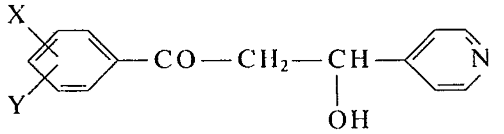

(II$_A$)

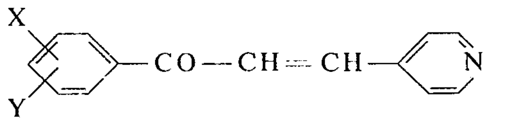

(II$_B$)

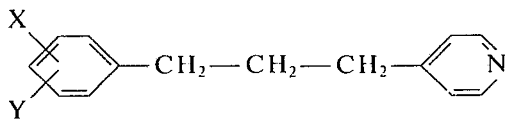

(II$_C$)

Cette hydrogénation catalytique est effectuée au sein d'un solvant inerte et en présence d'un catalyseur, à une température située entre 20 et 80° C et sous une pression d'hydrogène de 1 à 50 bars. Comme solvant on peut utiliser par exemple des alcools, tels que le méthanol ou l'éthanol, ou des acides tels que l'acide acétique. Comme catalyseur peuvent être mentionnés le nickel, la palladium, le rhodium, le ruthenium ou le platine.

Les composés de formule générale (I) pour lesquels A représente un groupe $CH_2$ et l'un au moins des substituants X et Y est un groupe $NH_2$ peuvent également être préparés par hydrogénation catalytique, dans des conditions identiques à celles employées pour l'hydrogénation des composés de formule (II), des composés de formule:

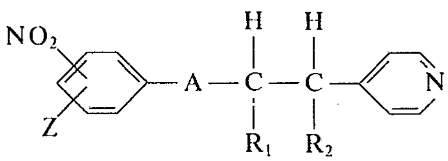

(III)

dans laquelle A est

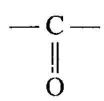

CHOH ou $CH_2$, $R_1$ et $R_2$ ont les significations indiquées précédemment et Z a les mêmes significations que X et Y dans la formule (I).

Les composés de formule (I) pour lesquels A représente le groupe CHOH peuvent être préparés par réduction des composés de formule (I) correspondants pour lesquels A représente le groupe CO. On peut utiliser à cet effet les procédés connus en soi permettant de transformer une cétone en alcool (cf. R. B. WAGNER et H. D. ZOOK, Synthetic Organic Chemistry, p. 149 — J. Wiley and Sons 1953). Il est avantageux d'utiliser comme agent réducteur un hydrure métallique tel que le borohydrure de sodium, de potassium ou de lithium, l'hydrure d'aluminium et de lithium, l'hydrure de bis (méthoxy-2 éthoxy) aluminium ou encore le diborane. Ces hydrures sont généralement utilisés en excès, au sein d'un solvant inerte tel que l'éther, le tétrahydrofuranne ou un hydrocarbure, à une température située entre 10° C et la température d'ébullition du solvant utilisé.

Les produits de formule générale (I) pour lesquels A représente le groupe CO peuvent être préparés par hydrogénation catalytique ménagée des produits de formule (II$_B$) ou de leurs sels. Il convient d'arrêter l'hydrogénation lorsque la quantité d'hydrogène théoriquement nécessaire à l'hydrogénation du groupe $-CH=CH-$ et du cycle pyridinique a été absorbée. On opère généralement au voisinage de la température ambiante, sous une pression d'hydrogène voisine de la pression atmosphérique, au sein d'un solvant inerte tel qu'un alcool (par exemple le methanol) ou l'éthanol) ou un acide (par exemple l'acide acétique). Comme catalyseurs peuvent être mentionnés le palladium, le rhodium, le ruthénium ou le platine.

Les composés de formule (I) pour lesquels A représente le groupe CO peuvent également être préparés, par une réaction d'acylation de Friedel et Crafts, en faisant réagir un dérivé benzénique de formule (IV) avec un dérivé pipéridinique de formule (V) et en hydrolysant le composé de formule (VI) ainsi formé, selon le schéma réactionnel suivant:

a) 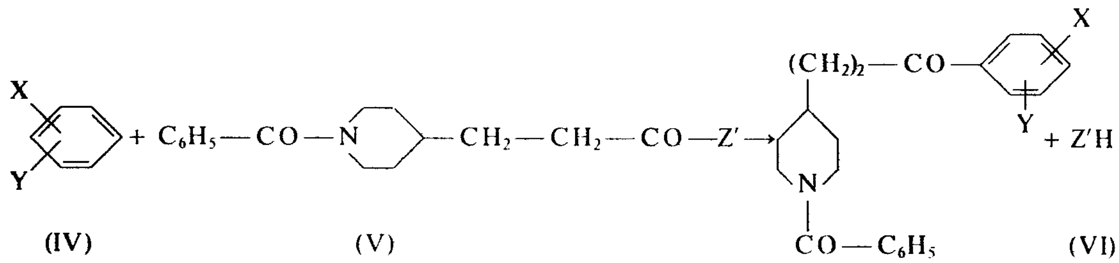

(IV) (V) (VI)

b) 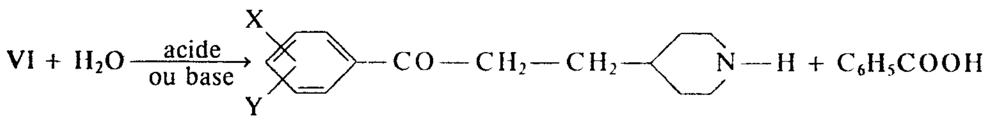


Dans la formule (V), Z′ désigne un groupe OH ou un atome d'halogène.

Pour réaliser la réaction a) on opère en présence des catalyseurs connus sous le nom de catalyseurs de Friedel et Crafts (halogénures métalliques, oxydes métalliques, iode, acides minéraux, etc...), dans des conditions telles que celles décrites dans »Friedel-Crafts and related reactions«, OLAH, vol. 3 (Inter-science 1964). Lorsque Z′ représente un atome de chlore on emploie avantageusement comme catalyseur le chlorure d'aluminium, en présence ou non d'un solvant, les solvants utilisés de préférence étant le sulfure de carbone ou le dichloro-1,2 éthane. Lorsque Z′ est un groupe OH on peut opérer par exemple en présence d'acide polyphosphorique, à une température voisine de 100° C.

La réaction d'hydrolyse b) est faite selon des procédés connus en soi (cf. par exemple »Hydrolysis of N-substituted amides« dans »Synthetic Organic Chemistry«, p. 678, R. B. WAGNER et H. D. ZOOK, J. Wiley and Sons 1953). On peut par exemple l'effecteur à l'aide d'une solution aqueuse, chauffée au reflux, d'un acide minéral tel que l'acide chlorhydrique ou l'acide sulfurique ou d'une base minérale telle que l'hydroxyde de sodium ou de potassium.

Suivant la nature des substituants X et Y le rendement de la réaction (a) varie ainsi que la position sur le cycle benzénique où se fait l'acylation de façon prépondérante (cf. J. MARCH, »Advanced Organic Chemistry Reactions, Mechanisms and Structure«, Mc. GRAW Hill 1968, p. 382 à 391). La méthode faisant appel à la réaction d'acylation de Friedel et Crafts est avantageuse dans le cas où X est un atome d'hydrogène ou d'halogène ou un groupe alkyle ou alcoxy et Y est un atome d'hydrogène ou un groupe identique à X, non situé en position para par rapport à X. Dans ce cas la réaction s'effectue avec de bons rendements et les produits obtenus portent la chaîne acyle en position para par rapport au groupement X.

Les mélanges réactionnels obtenus par les procédés décrits précédemment sont traités suivant des méthodes classiques, physiques (évaporation, extraction à l'aide d'un solvant, distillation, cristallisation, chromatographie, etc...) ou chimiques (formation de sel et régénération de la base, etc...) afin d'isoler le produit de formule (I) à l'état pur.

Les composés de formule (I) sous forme de bases libres peuvent éventuellement être transformés en sels d'addition avec un acide minéral ou organique par action d'un tel acide au sein d'un solvant approprié.

Certains composés répondant aux formules ($II_A$), ($II_B$) et ($II_C$) sont déjà connus. Le composé de formule ($II_A$) avec Y = H et X = OH en position 2 a été décrit par A. CORVAISIER (Bull. Soc. Chim. Fr. 1962, 528–534). Les composés de formule ($II_B$) avec X = Y = H et avec X = OH en position 2 et Y = H, $OCH_3$ en position 4, Cl ou Br en position 4 ou 5 ont été décrits par A. CORVAISIER (déjà cité), C. S. MARVEL et Coll. (J. Org. Chem. 1785 [1955]), A. C. ANNIGERI et Coll. (Monatsch. Chem. 96, 625 [1965]) et P. DEVITT et Coll. (J. Org. Chem. 26, 4941 [1961]). Les composés de formule ($II_C$) avec Y = H et X = H, $OCH_3$ en position 2, 3 ou 4, $CH_3$ en position 4, isopropyle en position 4, tert-butyle en position 4 ont été décrits par K. B. PRASAD et Coll. (déjà cité) et dans le DOS 2 456 947.

Les composés de formules ($II_A$) et ($II_B$) peuvent être préparés, selon un procédé général connu (cf. HOUBEN–WEYL, Methoden der organischen Chemie 7 [2b], 1483), en condensant le pyridine carbaldéhyde-4 avec une acétophénone de formule (VII) suivant le schéma réactionnel:

c) 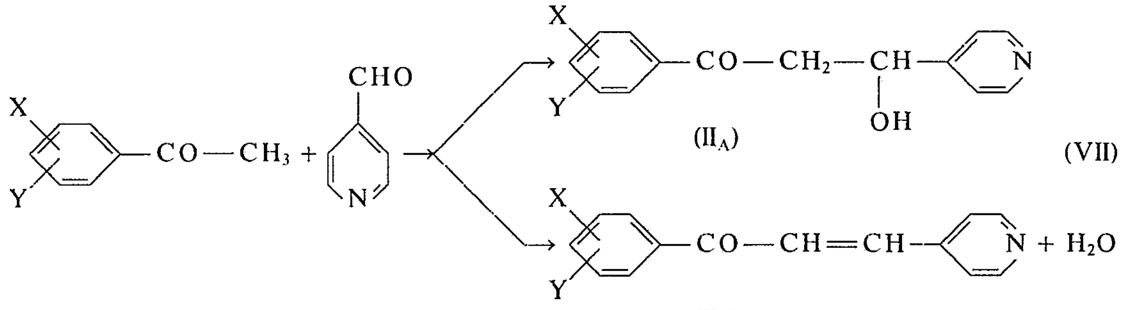

(II$_A$) (VII)

(II$_B$)

La réaction de condensation c) peut être réalisée au sein d'un alcool pur, tel que le méthanol ou l'éthanol, en présence d'une base telle que l'hydroxyde de tétraméthylammonium ou une résine basique (par exemple IRA-400), à température ambiante. On peut également opérer en présence d'hydroxyde de sodium dans un milieu hydroalcoolique. Suivant les conditions et suivant la nature des substituants X et Y on obtient soit le composé (II$_B$) soit le composé (II$_A$) soit encore un mélange des deux produits, que l'on peut ensuite séparer par cristallisation ou chromatographie.

Les composés de formule (II$_C$) peuvent être obtenus de manière générale par réduction non catalytique des dérivés pyridiniques de formule:

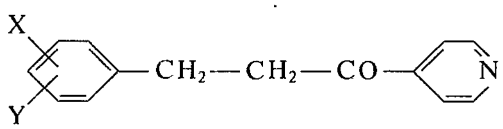

(VIII)

eux-mêmes obtenus par condensation de la cyano-4 pyridine avec le dérivé organo-magnésien approprié selon le procédé décrit par K. P. PRASAD et Coll., J. Chem. Soc. (C) 1969, 2134.

On utilise avantageusement, comme agent réducteur, l'hydrate d'hydrazine en présence d'un hydroxyde de métal alcalin tel que l'hydroxyde de sodium au sein d'un solvant tel qu'un alcool. On opère en général à température d'ébullition du solvant.

Les composés de formule (III), peuvent être préparés par nitration des composés de formule:

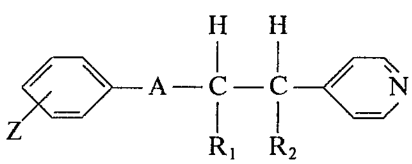

(IX)

dans laquelle A, R$_1$, R$_2$ et Z ont les mêmes significations que dans la formule (III).

Les exemples suivants illustrent l'invention sans la limiter. Les exemples 1 à 5 concernent la préparation des composés de formule (VI), les exemples 11 à 15 celle des composés de formule (II), les exemples 6 à 10 et 16 à 22 celle des composés de formule (I).

### Exemple 1

(benzoyl-1 pipéridyl-4)-3 (méthoxy-4 phényl)-1 propanone-1

On agite fortement 9,9 g d'anisole en solution dans 157 ml de sulfure de carbone avec 17 g de chlorure de (benzoyl-1 pipéridyl-4)-3 propionyle [préparé par action de 8,8 ml de chlorure de thionyle sur 15,7 g d'acide (benzoyl-1 pipéridyl-4)-3 propionique au sein du chloroforme]. Puis on ajoute, par portions, 20,2 g de chlorure d'aluminium finement divisé. La température monte progressivement à 35° C.

On chauffe ensuite à 50° C pendant 20 mn, puis le mélange réactionnel est jeté sur de la glace pilée et on extrait par deux fois 200 ml de chloroforme. Les phases organiques rassemblées sont lavées à l'eau, séchées sur sulfate de magnésium, puis concentrées par élimination du chloroforme. L'huile résiduaire obtenue est recristallisée dans 200 ml d'un mélange éther-éther de pétrole et les cristaux obtenus sont séchés à 65° C sous vide.

On obtient ainsi 16,7 g de (benzoyl-1 pipéridyl-4)-3 (méthoxy-4 phényl)-1 propanone-1.

Spectre R.M.N. du produit obtenu (solvant: deutérochloroforme; référence: tétraméthylsilane):

Les déplacements chimiques $\delta$ sont les suivants:

$OCH_3$ : 3,9 ppm (s)

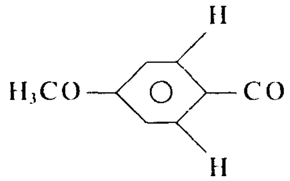

: 7,9 ppm (d)

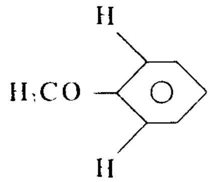

: 6,9 ppm (d)

L'acide (benzoyl-1 pipéridyl-4)-3 propionique utilisé comme produit de départ a été décrit par KOELSCH (J. Am. Chem. Soc. 1943, 65, 2460).

Exemple 2

(benzoyl-1 pipéridyl-4)-3 (flucro-4 phényl)-1 propanone-1

On opère comme à l'exemple 1 en partant de 8,8 g de fluorobenzène au lieu de 9,9 g d'anisole et de 20 g d'acide (benzoyl-1 pipéridyl-4)-3 propionique au lieu de 15,7 g. On obtient 25,5 g de (benzoyl-1 pipéridyl-4)-3 (fluoro-4 phényl)-1 propanone-1 brute sous forme d'une huile.

Exemple 3

(dichloro-3,4 phényl)-1 (benzoyl-1 pipéridyl-4)-3 propanone-1

On remplace dans l'exemple 1 les 9,9 g d'anisole et 15,7 g d'acide (benzoyl-1 pipéridyl-4)-3 propionique par respectivement 185 ml de dichloro-1,2 benzène et 35 g d'acide (benzoyl-1 pipéridyl-4)-3 propionique. La réaction est effectuée à 100°C et sans solvant. On obtient 51,1 g de (dichloro-3,4 phényl)-1 (benzoyl-1 pipéridyl-4)-3 propanone-1 brute sous forme d'une huile qui cristallise lentement.

Exemple 4

(chloro-4 phényl)-1 (benzoyl-1 pipéridyl-4)-3 propanone-1

On opère comme à l'exemple 1 en partant de 18 g de chlorobenzène au lieu de 9,9 g d'anisole, 350 ml de sulfure de carbone au lieu de 157 ml et 35 g d'acide (benzoyl-1 pipéridyl-4)-3 propionique au lieu de 15,7 g. On obtient 40,1 g de (chloro-4 phényl)-1 (benzoyl-1 pipéridyl-4)-3 propanone-1 brute sous forme d'une huile qui cristallise.

Exemple 5

(méthyl-4 phényl)-1 (benzoyl-1 pipéridyl-4)-3 propanone-1

On opère comme à l'exemple 4 en remplaçant les 18 g de chlorobenzène par 30 g de toluène. On obtient 34 g de (méthyl-4 phényl)-1 (benzoyl-1 pipéridyl-4)-3 propanone-1 brute sous forme d'une huile.

Exemple 6

[(méthoxy-4 phényl)-3 Propyl-1]-4 pipéridine

On chauffe pendant 20 mn à 140°C une solution contenant 16,7 g de (benzoyl-1 pipéridyl-4)-3 (méthoxy-4 phényl)-1 propanone-1, 45 ml de diéthylène glycol et 8,4 g d'hydrate d'hydrazine à 85%. La

solution jaune obtenue est refroidie à 100° C, puis on ajoute par portions 16 g d'hydroxyde de potassium en pastilles. On porte alors la température à 160° C pendant 4 heures, puis à 190° C pendant 2 heures. On refroidit le mélange et introduit 450 ml d'eau. On extrait deux fois par 150 ml d'acétate d'éthyle puis sèche la phase organique avec du sulfate de magnésium. Après filtration et concentration par élimination de l'acétate d'éthyle, on obtient une huile qui, acidifiée par une solution d'acide chlorhydrique dans l'acétone, fournit 10,2 g de chlorhydrate de [(méthoxy-4 phényl)-3 propyl-1]-4 pipéridine fondant à 145° C.

Analyse pour $C_{15}H_{23}NO$, HCl

| | | | |
|---|---|---|---|
| Calculé | % C 66,8 | % H 8,91 | % N 5,19 |
| Trouvé | % C 66,60 | % H 8,85 | % N 5,01 |

Exemple 7

[(fluoro-4 phényl)-3 propyl-1]-4 pipéridine

On opère comme à l'exemple 6 en partant de 24,2 g de (benzoyl-1 pipéridyl-4)-3 (fluoro-4 phényl)-1 propanone-1. On obtient 8,7 g de [(fluoro-4 phényl)-3 propyl-1]-4 pipéridine sous forme de chlorhydrate fondant à 142° C.

Analyse pour $C_{14}H_{20}NF$, HCl

| | | | |
|---|---|---|---|
| Calculé | % C 65,2 | % H 8,16 | % N 5,44 |
| Trouvé | % C 65,2 | % H 8,22 | % N 5,41 |

Exemple 8

[(chloro-4 phényl)-3 propyl-1]-4 pipéridine

On opère comme à l'exemple 6 en partant de 40,1 g de (benzoyl-1 pipéridyl-4)-3 (chloro-4 phényl)-1 propanone-1. On obtient 23,5 g d'une huile brune, qui fournit, après acidification par HCl et recristallisation, 19,6 g de [(chloro-4 phényl)-3 propyl-1]-4 pipéridine sous forme de chlorhydrate qui fond à 142° C.

Spectre R.M.N. du produit obtenu (solvant: $CDCl_3$; référence: tétraméthylsilane):

| | |
|---|---|
| aromatiques: | $\delta = 6,8$ à 7,4 ppm (m) |
| $CH_2-N$ et $CH_2-Ar$: | $\delta = 2,4$ à 3,3 ppm (m) |

Exemple 9

[(dichloro-3,4 phényl)-3 propyl-1]-4 pipéridine

On opère comme à l'exemple 6 en partant de 51,1 g de (benzoyl-1 pipéridyl-4)-3 (dichloro-3,4 phényl)-1 propanone-1. On obtient 15,3 g de [(dichloro-3,4 phényl)-3 propyl-1]-4 pipéridine sous forme de chlorhydrate fondant à 150° C.

Spectre R.M.N. du produit obtenu (solvant $CDCl_3$):

| | |
|---|---|
| aromatique: | $\delta = 6,8$ à 7,4 ppm (m) |
| $CH_2-N$ et $CH_2-Ar$: | $\delta = 2,4$ à 3,3 ppm (m) |

Exemple 10

[(méthyl-4 phényl)-3 propyl-1]-4 pipéridine

On opère comme à l'exemple 6 en partant de 34,2 g de (benzoyl-1 pipéridyl-4)-3 (méthyl-4 phényl)-1 propanone-1. On obtient 12 g de [(méthyl-4 phényl)-3 propyl-1]-4 pipéridine qui fond à 171° C.

Spectre R.M.N. du produit obtenu (solvant $CDCl_3$):

| | |
|---|---|
| aromatique: | $\delta = 7$ ppm (s) |
| $CH_3-Ar$: | $\delta = 2,3$ ppm (s) |
| $CH_2-N$ et $CH_2-Ar$: | $\delta = 2,3$ à 3,3 ppm (m) |

Exemple 11

(hydroxy-2 phényl)-1 (pyridyl-4)-3 hydroxy-3 propanone-1

Selon la méthode de CORVAISIER (Bull. Soc. Chim. Fr. 1962, 528), on ajoute, à une solution de 24 g d'hydroxy-2 acétophénone et de 21 g de pyridine carbaldéhyde-4 dans 130 ml d'éthanol absolu maintenue à température ambiante, 15 ml d'une solution aqueuse 11N d'hydroxyde de sodium. Un précipité abondant jaune se forme d'abord puis se dissout progressivement. Après deux heures à température ambiante, on amène à pH 1 au moyen d'acide chlorhydrique. Le précipité jaune obtenu est filtré, lavé à l'eau et séché. On obtient 27 g d'(hydroxy-2 phényl)-1 (pyridyl-4)-3 hydroxy-3 propanone-1 fondant à 150° C.

Spectre R.M.N. du produit obtenu (solvant: acide trifluoroacétique):

| | |
|---|---|
| $-CH_2(CHOH)$: | $\delta = 3{,}3$ ppm (d) |
| CHOH: | $\delta = 5{,}5$ ppm (t) |

Exemple 12

(amino-4 phényl)-1 (pyridyl-4)-3 propène-2 one-1

On opère comme à l'exemple 11 en partant de 1,35 g d'amino-4 acétophénone (au lieu de 24 g d'hydroxy-2 acétophénone) et de 2,14 g de pyridine carbaldéhyde-4 au lieu de 21 g et en employant une solution aqueuse 2,5N d'hydroxyde de sodium au lieu d'une solution 11N. On obtient 2,2 g d'(amino-4 phényl)-1 (pyridyl-4)-3 propène-2 one-1 qui fond à 207° C.

Spectre R.M.N. du produit obtenu (solvant: acide trifluoroacétique):

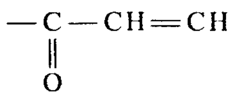

: $\delta$ = 7,4 ppm (d)
7,7 ppm (d)

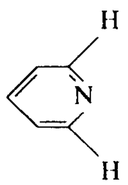

: $\delta$ = 8,6 ppm (d)

Exemple 13

(fluoro-4 phényl)-1 (pyridyl-4)-3 propène-2 one-1

En opérant comme à l'exemple 11 à partir de 2,9 g de fluoro-4 acétophénone et 3 g de pyridine carbaldéhyde-4 mais en utilisant une solution d'hydroxyde de tétraméthylammonium dans le méthanol au lieu de la solution aqueuse d'hydroxyde de sodium, on obtient la (fluoro-4 phényl)-1 (pyridyl-4)-3 propène-2 one-1 qui fond à 157° C.

Spectre R.M.N. du produit obtenu (solvant $CDCl_3$):

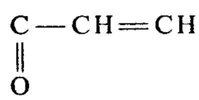

: $\delta$ = 7,6 ppm (s)

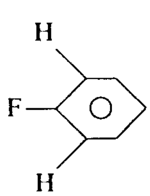

: $\delta$ = 7,2 ppm (t)

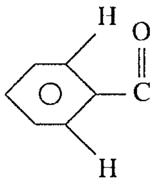

: δ = 8 ppm (d)

### Exemple 14

(amino-2 phényl)-1 (pyridyl-4)-3 propène-2 one-1

On opère comme à l'exemple 13 à partir de 4 g d'amino-2 acétophénone et de 6,42 g de pyridine carbaldéhyde-4 dans 14 ml de méthanol. On obtient 5,2 g d'un mélange 50/50 d'(amino-2 phényl)-1 (pyridyl-4)-3 hydroxy-3 propanone-1 et d'(amino-2 phényl)-1 (pyridyl-4)-3 propène-2 one-1. Les constituants de ce mélange sont séparés par chromatographie sur colonne de silice (éluant: mélange de 80 parties en volume d'acétate d'éthyle et 20 parties en volume de cyclohexane). On obtient 2,3 g d'(amino-2 phényl)-1 (pyridyl-4)-3 propène-2 one-1 qui fond à 168° C.

Spectre R.M.N. du produit obtenu (solvant $CDCl_3$):

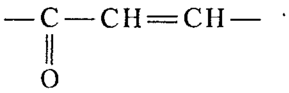

: δ = 7,2 ppm (d)
7,6 ppm (d)

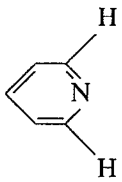

: δ = 8,6 ppm (d)

### Exemple 15

(méthoxy-2 phényl)-1 (pyridyl-4)-3 propène-2 one-1

On opère comme à l'exemple 11 en partant de 15 g de méthoxy-2 acétophénone et de 20,4 g de pyridine carbaldéhyde-4 dans 50 ml de méthanol et en employant 45 ml d'une solution aqueuse 2,5N d'hydroxyde de sodium à la place de la solution 11N. On obtient 7 g de (méthoxy-2 phényl)-1 (pyridyl-4)-3 propène-2 one-1 qui fond à 70° C.

Spectre R.M.N. du produit obtenu (solvant $CDCl_3$):

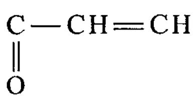

: δ = 7,6 ppm (s)

$OCH_3$ : δ = 3,95 ppm (s)

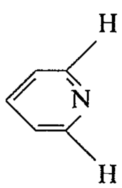

: δ = 8,6 ppm (d)

### Exemple 16

(phényl-3 propyl)-4 pipéridine

Une suspension bien agitée contenant 19,7 g de (phényl-3 propyl)-4 pyridine (préparée selon K. B. PRASAD et Coll., J. Chem. Soc. Ser. C, 1969, 2134) en solution dans 300 ml d'acide acétique et 2 g d'oxyde de platine ADAMS est maintenue, à température ambiante, sous une pression d'hydrogène correspondant à une surpression de 50 mm d'eau par rapport à la pression atmosphérique, jusqu'à cessation de l'absorption de gaz.

Le platine est ensuite séparé par filtration puis la solution acétique est concentrée. Le résidu est dilué par 300 ml d'eau, amené à pH 10 par addition de carbonate de potassium puis extrait par 2 fois 300 ml de chloroforme. Les fractions chloroformiques rassemblées sont séchées sur sulfate de magnésium puis concentrées. On obtient 19,2 g d'un produit impur, qui, après acidification par HCl et cristallisation dans l'acétone, fournit 18,8 g de (phényl-3 propyl)-4 pipéridine sous forme de chlorhydrate fondant à 167° C.

Analyse pour $C_{14}H_{21}N$, HCl

| | | | |
|---|---|---|---|
| Calculé | % C 70 | % H 9,2 | % N 5,85 |
| Trouvé | % C 70,15 | % H 9,21 | % N 5,69 |

Exemple 17

[(amino-4 phényl)-3 propyl]-4 pipéridine

En hydrogénant, selon le procédé de l'exemple 16, 29,7 g de [(nitro-4 phényl)-3 propyl]-4 pyridine, on obtient un mélange dont les constituants sont séparés par chromatographie sur colonne de silice (éluant: mélange de 90 parties en volume de chloroforme et 10 parties en volume de diéthylamine). Après acidification par HCl des fractions séparées, on obtient 1,8 g de chlorhydrate d'[(amino-4 phényl)-3 propyl]-4 pipéridine qui fond à 250° C.

Spectre R.M.N. du produit obtenu (solvant $CDCl_3$):

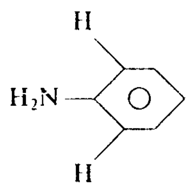 : $\delta$ = 6,8 ppm (d)

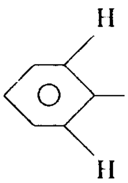 : $\delta$ = 7 ppm (d)

$CH_2$—Ar et $CH_2$—N : $\delta$ = 2,2 à 3,2 ppm (m)

La [(nitro-4 phényl)-3 propyl]-4 pyridine, produit de départ, est préparée comme suit:

Dans une solution refroidie à 5° C, sonstituée de 10,3 ml d'acide acétique pur et 18,6 ml d'acide sulfurique à 98%, on introduit lentement, goutte à goutte, 25 g de (phényl-3 propyl)-4 pyridine. Lorsque cette addition est terminée, on refroidit la solution obtenue à −15° C et coule, goutte à goutte, un mélange sulfonitrique constitué de 9 ml d'acide nitrique et 18,6 ml d'acide sulfurique à 98%. Pendant toute la durée de l'addition on maintient la température du milieu réactionnel en dessous de +10° C. L'addition est terminée en une heure. La solution jaune obtenue est jetée dans 600 g d'un mélange eau + glace. On amène à pH 11 par addition d'une solution aqueuse d'hydroxyde de potassium et extrait par deux fois 200 ml d'éther. Les phases éthérées rassemblées sont lavées à l'eau, séchées sur sulfate de magnésium et concentrées. On obtient 29,7 g de [(nitro-4 phényl)-3 propyl]-4 pyridine.

Spectre R.M.N. du produit obtenu (solvant $CDCl_3$):

$(CH_2)$—$CH_2$—$(CH_2)$ : $\delta$ = 2,1 ppm (m)

$CH_2$—Ar : $\delta$ = 2.8 ppm (m)

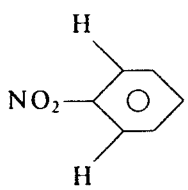 : $\delta$ = 8,1 ppm (d)

## Exemple 18

(acétylamino-2 phényl)-1 (pipérydyl-4)-3 propanone-1

Une suspension bien agitée constituée de 43,5 g d'(acétylamino-2 phényl)-1 (pyridyl-4)-3 propène-2 one-1, 4,3 g d'oxyde de platine ADAMS et 450 ml d'acide acétique est maintenue, à température ambiante, sous une pression d'hydrogène correspondant à une surpression de 50 mm d'eau par rapport à la pression atmosphérique, jusqu'à absorption de 8 équivalents d'hydrogène.

Le platine est ensuite séparé par filtration puis la solution acétique est concentrée. Le résidu est dilué par 500 ml d'eau, amené à pH 10 par une solution concentrée d'hydroxyde de sodium puis extrait par 3 fois 200 ml d'acétate d'éthyle. Les phases organiques rassemblées sont lavées par deux fois 50 ml d'eau, séchées sur sulfate de magnésium, puis concentrées.

On obtient ainsi 38,4 g d'un produit huileux brun qui, après acidification à pH 5 par une solution d'acide chlorhydrique dans l'éther, est transformé en chlorhydrate. La recristallisation du chlorhydrate brut dans l'éthanol fournit 26 g d'(acétylamino-2 phényl)-1 (pipéridyl-4)-3 propanone-1 sous forme de chlorhydrate fondant à 217° C.

Spectre R.M.N. du produit obtenu (solvant $CDCl_3$):

$-\underset{\underset{O}{\|}}{C}-NHCH_3$ : $\delta = 2{,}2$ ppm (s)

$CH_2-N$ et $-\underset{\underset{O}{\|}}{C}-CH_2$ : $\delta = 2{,}4$ à 3,2 ppm (m)

L'(acétylamino-2 phényl)-1 (pyridyl-4)-3 propène-2 one-1, produit de départ, est préparée comme suit:

On met en contact 45 g d'(amino-2 phényl)-1 (pyridyl-4)-3 propène-2 one-1 (produit de l'exemple 14) avec 13,5 ml d'anhydride acétique. Après 10 mn à 100° C, la solution obtenue est refroidie, diluée par 80 ml d'eau puis amenée lentement (en maintenant la température à 5° C) jusqu'à pH 6 par addition d'une solution concentrée d'hydroxyde de sodium. Après agitation pendant une heure, le précipité formé est filtré, lavé l'eau puis séché à 80° C sous vide. On obtient 53 g d'(acétylamino-2 phényl)-1 (pyridyl-4)-3 propène-2 one-1 qui fond à 130° C.

## Exemple 19

(méthoxy-4 phényl)-1 (pipéridyl-4)-3 propanone-1

Une suspension de 49 g de (benzoyl-1 pipéridyl-4)-3 (méthoxy-4 phényl)-1 propanone-1 dans 490 ml d'une solution aqueuse d'acide chlorhydrique 5N est chauffée 5 heures au reflux. Après refroidissement, on amène le pH à 3–4 par addition d'une lessive de soude, puis extrait par 500 ml d'éther l'acide benzoïque formé.

La phase aqueuse séparée est alcalinisée par addition d'une solution d'hydroxyde de sodium puis extraite par 250 ml d'acétate d'éthyle. La phase organique est séchée sur sulfate de magnésium puis concentrée. On obtient 39 g de produit brut qui, dilués dans 240 ml d'éthanol et acidifiés par 10 ml d'une solution 7N d'acide chlorhydrique dans l'éther, fournissent 12 g de (méthoxy-4 phényl)-1 (pipéridyl-4)-3 propanone-1 sous forme de chlorhydrate fondant à 168° C.

Analyse pour $C_{15}H_{21}NO_2$, HCl

| | | | |
|---|---|---|---|
| Calculé | % C 63,4 | % H 7,76 | % N 4,94 |
| Trouvé | % C 63,81 | % H 7,90 | % N 4,71 |

## Exemple 20

[(pipéridyl-4)-3 propyl]-2 phénol

Une solution bien agitée contenant 23 g d'(hydroxy-2 phényl)-1 (pyridyl-4)-3 hydroxy-3 propanone-1 (préparée selon CORVAISIER Bull. Soc. Chim. Fr. 1962, 528) en solution dans 340 ml d'acide acétique pur et 5 g de charbon palladié à 10% de palladium est maintenue à 80° C sous une pression d'hydrogène correspondant à une surpression de 50 ml d'eau par rapport à la pression atmosphérique, jusqu'à cessation de l'absorption du gaz.

Après refroidissement, le charbon palladié est séparé par filtration, puis la solution acétique est concentrée. On obtient 11,5 g d'une huile incolore qui, après recristallisation dans un mélange isopropanol-acétate d'éthyle, fournit 5,3 g de [(pipéridyl-4)-3 propyl]-2 phénol qui fond à 152° C.

Analyse pour $C_{14}H_{21}NO$

| | | | |
|---|---|---|---|
| Calculé | % C 76,71 | % H 9,59 | % N 6,39 |
| Trouvé | % C 76,49 | % H 9,51 | % N 6,42 |

## Exemple 21

### (fluoro-4 phényl)-1 (pipéridyl-4)-3 propanone-1

On opère comme à l'exemple 19, en partant de 12 g de (benzoyl-1 pipéridyl-4)-3 (fluoro-4 phényl)-1 propanone-1. Après acidification du produit brut obtenu par une solution d'acide chlorhydrique dans l'éther, on obtient 6,5 g de chlorhydrate de (fluoro-4 phényl)-1 (pipéridyl-4)-3 propanone-1 qui fond à 188° C.

Spectre R.M.N. du produit obtenu (solvant $CDCl_3$):

$CH_2$—N et 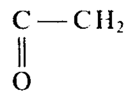 : $\delta$ = 2,5 à 3,6 ppm (m)

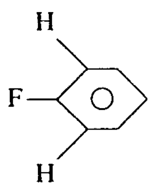 : $\delta$ = 7,2 ppm (m)

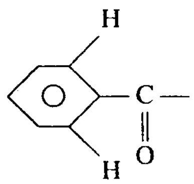 : $\delta$ = 8 ppm (m)

## Exemple 22

### (méthoxy-4 phényl)-1 (pipéridyl-4)-3 propanol-1

A une suspension de 10,2 g de (méthoxy-4 phényl)-1 (pipéridyl-4)-3 propanone-1 dans 130 ml de tétrahydrofuranne sec, maintenue à 5° C et sous atmosphère d'azote, on ajoute, par petites portions, 2,8 g d'hydrure d'aluminium et de lithium. Lorsque l'addition est terminée, le mélange réactionnel est porté à 60° C pendant 5 heures puis refroidi à 0° C. On ajoute alors très lentement, en maintenant cette température, 25,2 ml d'eau. Les produits minéraux sont filtrés et lavés par deux fois 50 ml de chlorure de méthylène chaud. Les solutions organiques rassemblées sont concentrées par évaporation du solvant. L'huile obtenue est mise en solution dans 150 ml d'isopropanol et traitée par 7,4 g d'acide fumarique. On obtient finalement ainsi 7,8 g de (méthoxy-4 phényl)-1 (pipéridyl-4)-3 propanol-1 sous forme de son fumarate.

Analyse pour 3 ($C_{15}H_{23}NO_2$), 2($C_4H_4O_4$)

| | | | |
|---|---|---|---|
| Calculé | % C 65 | % H 7,86 | % N 4,29 |
| Trouvé | % C 64,8 | % H 7,92 | % N 4,19 |

## Propriétés pharmacologiques

### 1. Activité antidépressive

On sait que les produits antidépresseurs actuellement connus possèdent la propriété d'inhiber la recapture, par les neurones, des monoamines cérébrales. Ils agissent à la fois sur la sérotonine ou hydroxy-5 tryptamine (5HT) et sur les catécholamines, notamment sur la noradrénaline (NA) et présentent divers effets secondaires, en particulier une cardiotoxicité.

Un moyen d'obtenir des antidépresseurs présentant moins d'effet secondaires que les

médicaments actuellement utilisés consiste à rechercher des produits agissant spécifiquement sur la sérotonine (J. BUUSLASSEN et Coll., Europ. J. Pharmacol. 33, 108 [1975]).

L'activité des produits de formule (I) a été démontrée à l'aide du test d'inhibition de la recapture des monoamines cérébrales par des synaptcsomes de cerveau de rat, selon la méthode de KANNENGIESSER et Coll. (Biochem. Pharmacol. 22, 73, 1973). Les résultats sont exprimés par une dose inhibitrice 50% ($I_{50}$) qui représente la dose de produit, en micromole par litre, diminuant de 50% la recapture du neuromédiateur considéré dans les régions spécifiques (hypothalamus, medulla + pons) où prédominent les neurones correspondants.

A titre d'exemple, on obtient les résultats suivants:

Tableau I

| Produits | $I_{50}$ (µM/l) NA (hypothalamus) | 5HT (medulla+pons) |
|---|---|---|
| Exemple 6 | 2.2 | 0,02 |
| Exemple 7 | 3 | 0,01 |
| Exemple 8 | 0,9 | 0,09 |
| Exemple 9 | 1,3 | 0,18 |
| Exemple 10 | 0,3 | 0,16 |
| Exemple 16 | 0,24 | 0,002 |
| Imipramine | 0,5 | 0,12 |

Le tableau I montre que les composés de formule (I) sont de puissants inhibiteurs de recapture de la sérotonine. Ils sont souvent plus actifs et surtout beaucoup plus sélectifs que l'imipramine (produit de référence). Ils inhibent spécifiquement la recapture de la sérotonine.

## 2. Activité anxiolytique

L'activité anxiolytique des benzodiazépines est bien connue. La présence de récepteurs spécifiques des benzodiazépines dans les membranes de cerveau de rat est également bien établie (SQUIRES et Coll., Nature 266, 732 [1977]) et il existe une bonne corrélation entre le degré d'affinité des benzodiazépines pour les sites de liaison récepteurs et les effets pharmacodynamiques observés chez l'animal et chez l'homme.

Cette affinité est mesurée par l'aptitude des produits à déplacer le diazépam tritié ($^3$H-diazépam) de son site de liaison et est exprimée par une valeur $K_i$, en micromoles, qui est calculée par la formule:

$$K_i = I_{50}\ [(1 + C)\ K_D]$$

dans laquelle C représente la concentration de $^3$H-diazépam, $K_D$ une constante d'affinité égale à 2,74 µm et $I_{50}$ la concentration nécessaire pour obtenir une inhibition de 50% de la liaison du $^3$H-diazépam.

Jusqu'à jour l'affinité des benzodiazépines pour leurs récepteurs cérébraux s'est avérée une caractéristique exclusive de cette série chimique. En effet aucun autre médicament agissant par ailleurs sur le système nerveux central ne s'est montré capable de déplacer, de manière significative, le diazépam de ses sites de liaison (BRAESTRUP et Coll., Eur. J. Pharmacol. 48, 263 [1978]).

Les produits de formule (I), bien que de structure différente de celle des benzodiazépines, déplacent le $^3$H-diazépam de ses sites de liaison, comme le montrent les résultats du tableau II ci-dessous:

Tableau II

| Produits | Ki (µM) |
|---|---|
| Exemple 6 | 19,5 |
| Exemple 7 | 45 |
| Exemple 8 | 36 |
| Exemple 10 | 42 |
| Exemple 17 | 36 |
| Exemple 20 | 29 |
| Exemple 19 | 6,5 |
| Exemple 22 | 32 |
| Imipramine | inactive |

Les résultats du tableau II ont été obtenus en testant les produits selon la méthode de H. MOHLER et Coll., Life Sciences 20, 2101 (1977).

Les produits de formule (I) sont donc non seulement des antidépresseurs mais aussi des drogues anxiolytiques. Le mécanisme d'action de ces composés (action spécifique sur la recapture de la sérotonine et affinité pour les récepteurs cérébraux des benzodiazépines) leur confère un profil de psychotrope entièrement original.

3. Activité antiarythmique

L'activité antiarythmique des composés de formule (I) a été démontrée à l'aide du test à l'aconitine chez le rat.

Le principe de la technique repose sur le temps d'induction des arythmies ventriculaires provoquées par l'aconitine en perfusion lente chez le rat. Une substance antiarythmique retarde l'apparition des arythmies et ce délai est proportionnel à l'activité de la molécule.

On utilise des groupes de 5 rats mâles. Une anesthésie individuelle est réalisée (uréthane 10%: 1 g/kg/ip) pour permettre une cathétérisation de la veine du pénis. L'électrocardiogramme est enregistré. Au temps T=0 la substance étudiée est injectée sous forme d'une solution aqueuse, à raison de 2,5 ml de solution par kg en 30 secondes. Au temps T=90 secondes, soit 1 minute après la fin de l'introduction, l'aconitine est perfusée à raison de 20 µg/1 min, jusqu'à l'apparition d'extra systoles supra-ventriculaires. Le temps de perfusion de l'aconitine est noté.

On exprime les résultats en $DE_{50}$, dose en mg/kg qui, par rapport aux animaux témoins, augmente de 50% le temps de perfusion de l'aconitine.

Les produits de formule (I) présentent de remarquables propriétés antiarythmiques, comme le montrent les résultats du tableau ci-après donnés à titre d'exemple.

Tableau III

| Produits | $DE_{50}$(i.v.)mg/kg |
|---|---|
| Exemple 16 | 2,5 |
| Exemple 20 | 1,2 |
| Quinidine (produit de référence) | 4 |

Propriétés toxicologiques

Les toxicités aiguës des composés de formule (I) ont été déterminés chez la souris mâle $CD_1$ (Charles RIVER) par voie orale. Les $DL_{50}$ ont été calculées, après 3 joirs d'observations, par la méthode cumulative de J. J. REED et H. MUENCH (Amer. J. Hyg. 27, 493 [1938]).

Les produits de formule (I) se comportent comme des substances peu toxiques chez la souris, comme le montrent les $DL_{50}$ rassemblés, à titre d'exemple, dans le tableau suivant:

Tableau IV

| Produits | Toxicité aiguë chez la souris (p.o.) $DL_{50}$ (en mg/kg) |
|---|---|
| Exemple 6 | 400 |
| Exemple 7 | 225 |
| Exemple 8 | 235 |
| Exemple 9 | 600 |
| Exemple 10 | 400 |
| Exemple 16 | 200 |
| Exemple 19 | 200 |
| Exemple 22 | 800 |

Utilisation thérapeutique

Les composés de formule (I) et leurs sels pharmaceutiquement acceptables peuvent être utilisés en thérapeutique humaine sous forme de comprimés, capsules, gélules, suppositoires, solutions ingérables ou injectables, etc... pour le traitement de divers troubles psychiques à composante dépressive et anxieuse et pour le traitement des arythmies.

La posologie dépend des effets recherchés et de la voie d'administration utilisée. Par exemple, par voie orale, elle peut être comprise entre 5 et 250 mg de substance active par jour, avec des doses unitaires allant de 1 à 50 mg.

**Revendications de brevet**

1. Composés de formule:

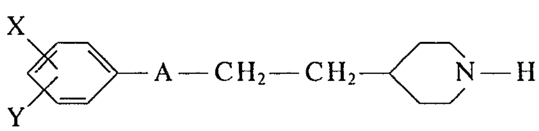

(I)

dans laquelle X et Y sont identiques ou différents et représentent des atomes d'hydrogène ou d'halogène ou des groupes alkyle ayant 1 à 4 atomes de carbone, alcoxy ayant 1 à 4 atomes de carbone, alkylthio ayant 1 à 4 atomes de carbone, trifluorométhyle, hydroxy, amino, monoalkylamino ayant 1 à 4 atomes de carbone, ou amino substitué par un groupe alkylsulfonyle ayant 1 à 4 atomes de carbone, par un groupe alkylcarbonyle ayant 1 à 5 atomes de carbone ou par un groupe benzoyle, et A représente un

groupe $\underset{\underset{O}{\|}}{-C-}$   CHOH   ou   $CH_2$

et leurs sels avec un acide pharmaceutiquement acceptable, utilisables dans le traitement de l'arythmie et des états de dépression et d'anxiété.

2. Composé selon la revendication 1 utilisable dans le traitement de l'arythmie et des états de dépression et d'anxiété, caractérisé en ce qu'il consiste en la (phényl-3 propyl)-4 pipéridine ou un de ses sels avec un acide pharmaceutiquement acceptable.

3. Composés chimiques répondant à la formule (I) de la revendication 1 dans lesquels A est un

groupe 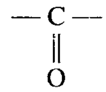 ou CHOH

et X et Y ont les mêmes significations que dans la revendication 1.

4. Composés chimiques répondant à la formule (I) de la revendication 1 dans lesquels A est un groupe $CH_2$, Y est un atome d'hydrogène ou d'halogène ou un groupe alkyle ayant 1 à 4 atomes de carbone, alcoxy ayant 1 à 4 atomes de carbone, alkylthio ayant 1 à 4 atomes de carbone, trifluorométhyle, hydroxy, amino, monoalkylamino ayant 1 à 4 atomes de carbone, ou amino substitué par un groupe alkylsulfonyle ayant 1 à 4 atomes de carbone, par un groupe alkylcarbonyle ayant 1 à 5 atomes de carbone ou par un groupe benzoyle et X est un atome d'halogène ou un groupe alkylthio ayant 1 à 4 atomes de carbone, alcoxy ayant 2 à 4 atomes de carbone, trifluorométhyle, hydroxy, amino, monoalkylamino ayant 1 à 4 atomes de carbone ou amino substitué par un groupe alkylsulfonyle ayant 1 à 4 atomes de carbone, par un groupe alkylcarbonyle ayant 1 à 5 atomes de carbone ou par un groupe benzoyle.

5. Procédé de préparation des composés selon la revendication 3 dans lesquels A est un groupe CHOH caractérisé en ce qu'il consiste à réduire les composés correspondants selon la revendication 3 dans lesquels A est un groupe

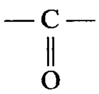

6. Procédé de préparation des composés selon la revendication 3 dans lesquels A est un groupe

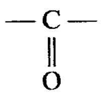

caractérisé en ce qu'il consiste à hydrogéner catalytiquement de façon ménagée les composés de formule:

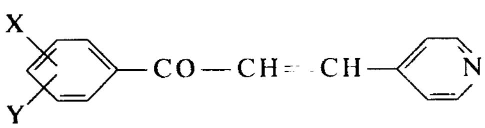

($II_B$)

dans laquelle X et Y ont les mêmes significations que dans la revendication 3, ou leurs sels.

7. Procédé de préparation des composés selon la revendication 3 dans lesquels A est un groupe

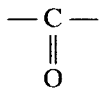

caractérisé en ce qu'il consiste à faire réagir, en présence d'un catalyseur de Friedel et Crafts, un dérivé benzénique de formule:

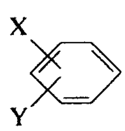

(IV)

dans laquelle X et Y ont les mêmes significations que dans la revendication 3, avec un dérivé pipéridinique de formule:

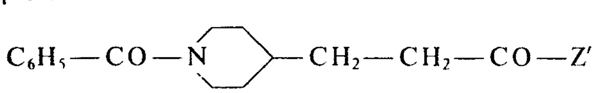

(V)

dans laquelle Z' est un groupe OH ou un atome d'halogène, et à hydrolyser le composé de formule:

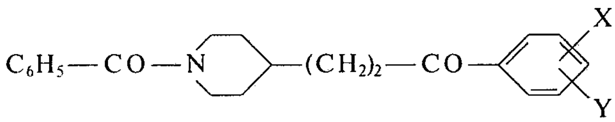 (VI)

ainsi formé.

8. Procédé de préparation des composés selon la revendication 4, caractérisé en ce qu'il consiste à réduire les composés correspondants de formule (I) dans lesquels A est un groupe

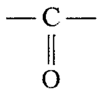

ou leurs dérivés N-benzoylés.

9. Procédé de préparation des composés selon la revendication 4, caractérisé en ce qu'il consiste à hydrogéner catalytiquement les composés pyridiniques de formule:

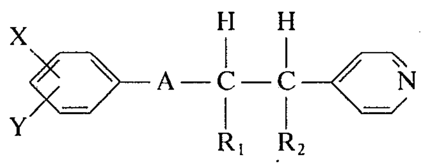 (II)

dans laquelle X et Y ont les significations indiquées à la revendication 4, A est un groupe

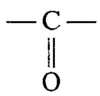

CHOH ou $CH_2$,

$R_1$ est un atome d'hydrogène et $R_2$ est un atome d'hydrogène ou un groupe OH, $R_1$ et $R_2$ pouvant en outre former ensemble une liaison simple.

10. Procédé de préparation des composés selon la revendication 4 dans lesquels l'un au moins des substituants X et Y est un groupe $NH_2$, caractérisé en ce qu'il consiste à hydrogéner catalytiquement les composés de formule:

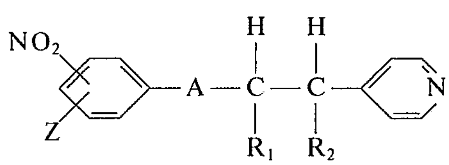 (III)

dans laquelle A est un groupe

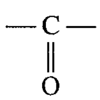

CHOH ou $CH_2$,

$R_1$ est un atome d'hydrogène, $R_2$ est un atome d'hydrogène ou un groupe OH, $R_1$ et $R_2$ pouvant en outre former ensemble une liaison simple, et Z a les significations indiquées pour Y à la revendication 4.

**Patentansprüche**

1. Verbindungen der Formel

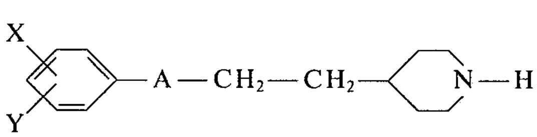 (I)

in der X und Y gleich oder verschieden sind und Wasserstoffatome, Halogenatome oder Alkylgruppen mit 1 bis 4 Kohlenstoffatomen, Alkoxygruppen mit 1 bis 4 Kohlenstoffatomen, Alkylthiogruppen mit 1 bis 4 Kohlenstoffatomen, Trifluormethyl, Hydroxy, Amino, Monoalkylamino mit 1 bis 4 Kohlenstoffatomen oder durch eine Alkylsulfonylgruppe mit 1 bis 4 Kohlenstoffatomen, durch eine Alkylcarbonylgruppe mit 1 bis 5 Kohlenstoffatomen oder durch eine Benzoylgruppe substituiertes Amino und A eine Gruppe

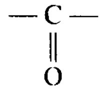

CHOH oder $CH_2$ bedeuten, und deren Salze mit einer pharmazeutisch verträglichen Säure, anwendbar bei der Behandlung von Arythmien, Depressions- und Angstzuständen.

2. Verbindung nach Anspruch 1, anwendbar bei der Behandlung von Arythmien, Depressions- und Angstzuständen, dadurch gekennzeichnet, daß sie aus 4-(3-Phenylpropyl)-piperidin oder eines seiner Salze mit einer pharmazeutisch verträglichen Säure besteht.

3. Chemische Verbindungen gemäß Formel (I) des Anspruchs 1, in denen A eine Gruppe

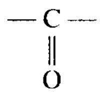

oder CHOH ist und X und Y die gleichen Bedeutungen wie in Anspruch 1 aufweisen.

4. Chemische Verbindungen gemäß Formel (I) des Anspruchs 1, in denen A eine $CH_2$-Gruppe, Y ein Wasserstoff- oder Halogenatom oder eine Alkylgruppe mit 1 bis 4 Kohlenstoffatomen, Alkoxy mit 1 bis 4 Kohlenstoffatomen, Alkylthio mit 1 bis 4 Kohlenstoffatomen, Trifluormethyl, Hydroxy, Amino, Monoalkylamino mit 1 bis 4 Kohlenstoffatomen oder durch eine Alkylsulfonylgruppe mit 1 bis 4 Kohlenstoffatomen, eine Alkylcarbonylgruppe mit 1 bis 5 Kohlenstoffatomen oder eine Benzoylgruppe substituiertes Amino und X ein Halogenatom oder eine Alkylthiogruppe mit 1 bis 4 Kohlenstoffatomen, Alkoxy mit 2 bis 4 Kohlenstoffatomen, Trifluormethyl, Hydroxy, Amino, Monoalkylamino mit 1 bis 4 Kohlenstoffatomen oder durch eine Alkylsulfonylgruppe mit 1 bis 4 Kohlenstoffatomen, eine Alkylcarbonylgruppe mit 1 bis 5 Kohlenstoffatomen oder durch eine Benzoylgruppe substituiertes Amino bedeuten.

5. Verfahren zur Herstellung von Verbindungen gemäß Anspruch 3, in denen A eine Gruppe CHOH ist, dadurch gekennzeichnet, daß man entsprechende Verbindungen gemäß Anspruch 3, in denen A eine Gruppe

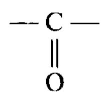

ist, reduziert.

6. Verfahren zur Herstellung von Verbindungen gemäß Anspruch 3, in denen A eine

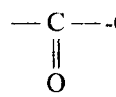 -Gruppe

ist, dadurch gekennzeichnet, daß man auf schonende Weise Verbindungen der Formel katalytisch hydriert:

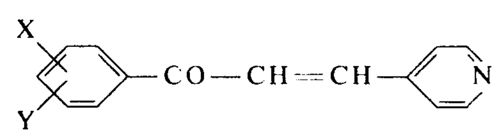 ($II_B$)

in der X und Y die gleichen Bedeutungen wie in Anspruch 3 haben, oder deren Salze.

7. Verfahren zur Herstellung von Verbindungen nach Anspruch 3, in denen A eine Gruppe

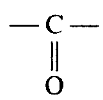

bedeutet, dadurch gekennzeichnet, daß man in Gegenwart eines Friedel-Crafts-Katalysators ein Benzolderivat der Formel

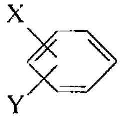

(IV)

in der X und Y die gleichen Bedeutungen wie in Anspruch 3 haben, mit einem Piperidinderivat der Formel reagieren läßt:

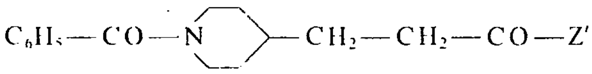

(V)

in der Z' eine OH-Gruppe oder ein Halogenatom ist und man die auf diese Weise erhaltene Verbindung der Formel

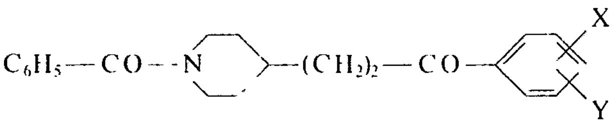

(VI)

hydrolysiert.

**8. Verfahren zur Herstellung von Verbindungen nach Anspruch 4, dadurch gekennzeichnet, daß man die entsprechenden Verbindungen der Formel (I), in den A eine**

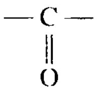 -Gruppe

**ist, oder ihre N-Benzoylderivate reduziert.**

**9. Verfahren zur Herstellung von Verbindungen nach Anspruch 4, dadurch gekennzeichnet, daß man Pyridinverbindungen der Formel**

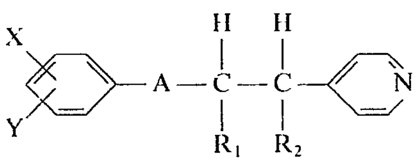

(II)

katalytisch hydriert, in der X und Y die gleichen Bedeutungen wie Anspruch 4 haben, A eine

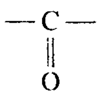 CHOH oder $CH_2$-Gruppe

**ist, $R_1$ ein Wasserstoffatom und $R_2$ ein Wasserstoffatom oder eine OH Gruppe bedeuten, und $R_1$ und $R_2$ außerdem zusammen eine Einfachbildung darstellen können.**

**10. Verfahren zur Herstellung von Verbindungen nach Anspruch 4, in denen mindestens einer der Substituenten X und Y eine $NH_2$-Gruppe bedeutet, dadurch gekennzeichnet, daß man Verbindungen der Formel**

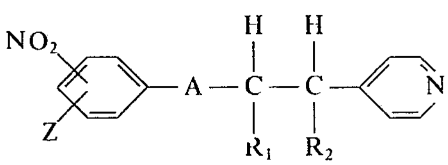

(III)

katalytisch hydriert, in der A eine

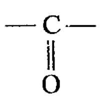 CHOH oder $CH_2$-Gruppe ist,

**$R_1$ ein Wasserstoffatom bedeutet, $R_2$ ein Wasserstoffatom oder eine OH-Gruppe ist, $R_1$ und $R_2$ außerdem zusammen eine Einfachbindung darstellen können und Z die für Y in Anspruch 4 angegebenen Bedeutungen hat.**

**Claims**

1. Compounds of the formula:

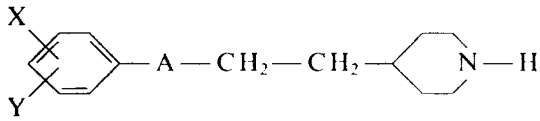

(I)

in which X and Y are the same or different and represent hydrogen or halogen atoms or alkyl groups having 1 to 4 carbon atoms, alkoxy groups having 1 to 4 carbon atoms, alkylthio groups having 1 to 4 carbon atoms, trifluoromethyl, hydroxy, amino, monoalkylamino groups having 1 to 4 carbon atoms, or amino groups substituted by an alkylsulphonyl group having 1 to 4 carbon atoms, by an alkylcarbonyl group having 1 to 5 carbon atoms or by a benzoyl group, and A represents a

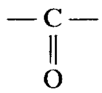 CHOH or $CH_2$ group,

and their salts with a pharmaceutically acceptable acid, for use in the treatment of arrythmia and states of depression and anxiety.

2. Compound according to claim 1, for use in the treatment of arrythmia and states of depression and anxiety, characterised in that it consists of 4-(3-phenyl-propyl)-piperidine or one of its salts with a pharmaceutically acceptable acid.

3. Chemical compounds corresponding to the formula (I) of claim 1, in which A is a

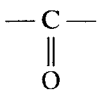 or CHOH group

and X and Y have the same signifiance as in claim 1.

4. Chemical compounds corresponding to the formula (I) of claim 1, in which A is a $CH_2$ group, Y is a hydrogen or halogen atom or an alkyl group having 1 to 4 carbon atoms, an alkoxy group having 1 to 4 carbon atoms, an alkylthio group having 1 to 4 carbon atoms, trifluoromethyl, hydroxy, amino, a monoalkylamino group having 1 to 4 carbon atoms, or an amino group substituted by an alkylsulphonyl group having 1 to 4 carbon atoms, by an alkylcarbonyl group having 1 to 5 carbon atoms or by a benzoyl group and X is a halogen atom or an alkylthio group having 1 to 4 carbon atoms, an alkoxy group having 2 to 4 carbon atoms, trifluoromethyl, hydroxy, amino, a monoalkylamino group having 1 to 4 carbon atoms or an amino group substituted by an alkylsulphonyl group having 1 to 4 carbon atoms, by an alkylcarbonyl group having 1 to 5 carbon atoms or by a benzoyl group.

5. Process for the preparation of the compounds according to claim 3 in which A is a CHOH group, characterised in that it consists in reducing the corresponding compounds according to claim 3 in which A is a group

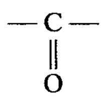

6. Process for the preparation of the compounds according to claim 3 in which A is a

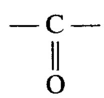 group,

characterised in that it consists in carrying on the partial catalytic hydrogenation of the compounds of the formula:

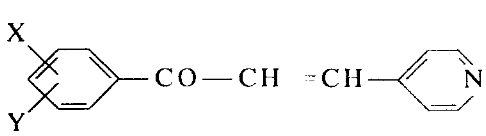

($II_B$)

in which X and Y have the same significance as in claim 3, or their salts.

7. Process for the preparation of the compounds according to claim 3 in which A is a

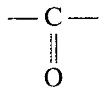 group,

characterised in that it consists in reacting, in the presence of a Friedel-Crafts catalyst, a benzene derivative of the formula:

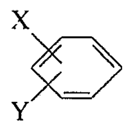 (IV)

in which X and Y have the same significance as in claim 3, with a piperidine derivative of the formula:

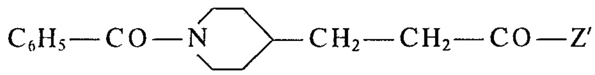 (V)

in which Z′ is an OH group or a halogen atom, and hydrolysing the compound of formula:

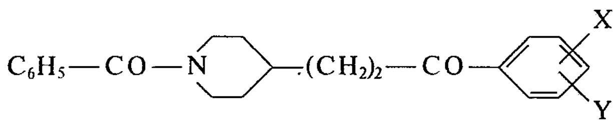 (VI)

thus formed.

8. Process for the preparation of the compounds according to claim 4, characterised in that it consists in reducing the corresponding compounds of formula (I) in which A is a

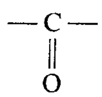 group

or their N-benzoyl derivatives.

9. Process for the preparation of the compounds according to claim 4, characterised in that it consists in catalytically hydrogenating the pyridine compounds of the formula:

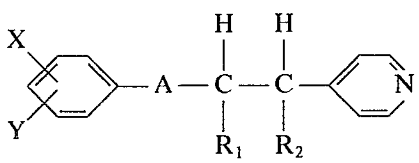 (II)

in which X and Y have the significance indicated in claim 4, A is a

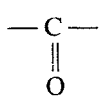 CHOH or $CH_2$ group,

$R_1$ is a hydrogen atom and $R_2$ is a hydrogen atom or an OH group, $R_1$ and $R_2$ also being able to form together a single bond.

10. Process for the preparation of the compounds according to claim 4 in which one at least of the substituents X and Y is an $NH_2$ group, characterised in that it consists in catalytically hydrogenating the compounds of the formula:

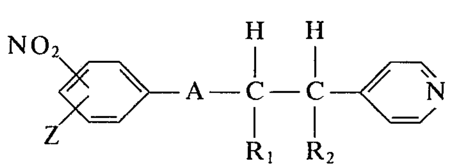 (III)

in which A is a

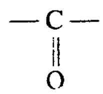 CHOH or $CH_2$ group,

$R_1$ is a hydrogen atom, $R_2$ is a hydrogen atom or an OH group, $R_1$ and $R_2$ being also able to form together a single bond, and Z has the significance indicated for Y in claim 4.